# EUROPEAN PATENT APPLICATION

(11) **EP 4 435 568 A1**
(43) Date of publication of application: **25.09.2024**
(21) Application number: 24164666.0
(22) Date of filing: 19.03.2024
(51) Int. Cl.: G06F 3/01, A61B 5/024, A61B 5/00

(54) **UTILIZING COINCIDENTAL MOTION INDUCED SIGNALS IN PHOTOPLETHYSMOGRAPHY FOR GESTURE DETECTION**

(30) Priority: 20.03.2023 US 202363491194 P; 12.03.2024 US 202418602671
(71) Applicant: Meta Platforms Technologies, LLC, Menlo Park, CA 94025 (US)
(72) Inventor: Korhonen, Ilkka, Menlo Park (US); Rajbhandary, Paurakh, Menlo Park (US)
(74) Representative: Murgitroyd & Company

(57) **Abstract**

Techniques for utilizing motion artifacts in photoplethysmography (PPG) signals are provided. In some embodiments, the techniques involve obtaining a set of PPG signals, the set of PPG signals acquired using one or more light emitters and one or more light detectors disposed in or on a portion of a wearable device. In some embodiments, the techniques involve separating the set of PPG signals into at least a physiological portion of the set of PPG signals and a motion artifact portion of the set of PPG signals. In some embodiments, the techniques involve identifying at least one hand or finger gesture of a wearer of the wearable device based at least in part on the motion artifact portion of the set of PPG signals.

## Description

### TECHNICAL FIELD

The present disclosure is directed to techniques for utilizing photoplethysmography motion artifacts for gesture detection.

### BACKGROUND

It may be useful for augmented reality (AR) and/or virtual reality (VR) implementations to identify hand or finger gestures made by a wearer of an AR/VR headset. For example, such gestures may be utilized to control functionality in an AR/VR world, such as by controlling user selection of various menu items, modifying visual or audio content based on the gesture, to draw patterns using hand movements, etc. However, it can be difficult to identify hand or finger gestures accurately.

### SUMMARY

Disclosed herein are techniques for utilizing photoplethysmography motion artifacts for gesture detection.

The present invention provides a method and a wrist-worn wearable device for utilizing motion artifacts in photoplethysmography (PPG) signals as defined in the appended claims.

In accordance with a first aspect, there is provided a method for utilizing motion artifacts in photoplethysmography (PPG) signals, the method comprising:
obtaining a set of PPG signals, the set of PPG signals acquired using one or more light emitters and one or more light detectors disposed in or on a portion of a wearable device;
separating the set of PPG signals into at least a physiological portion of the set of PPG signals and a motion artifact portion of the set of PPG signals;
identifying at least one hand or finger gesture of a wearer of the wearable device based at least in part on the motion artifact portion of the set of PPG signals.

The at least one hand or finger gesture may comprise at least one of: motion of an individual finger; motion of two or more fingers; a fist-closing gesture; a hand-waving gesture; or any combination thereof.

The at least one hand or finger gesture may be used to control functionality of an augmented reality (AR) or virtual reality (VR) headset paired with the wearable device.

The method may further comprise, prior to separating the set of PPG signals into at least the physiological portion of the set of PPG signals and the motion artifact portion of the set of PPG signals, determining that the set of PPG signals comprises the motion artifacts.

Determining that the set of PPG signals comprises the motion artifacts may be based on at least one of: a perfusion index at two different wavelengths of light of the set of PPG signals; a periodicity in the set of PPG signals; a skewness of the set of PPG signals; correlation of the set of PPG signals to a ground truth PPG signal without motion artifact; or any combination thereof.

Separating the set of PPG signals into at least the physiological portion of the set of PPG signals and the motion artifact portion of the set of PPG signals may comprise using an adaptive filter.

The method may further comprise separating the motion artifact portion of the set of PPG signals into a macromotion portion representing motion of multiple fingers and a micromotion portion representing motion of individual fingers.

Identifying the at least one hand or finger gesture may comprise applying a trained classifier to at least the motion artifact portion of the set of PPG signals.

Identifying the at least one hand or finger gesture may comprise utilizing at least one of: sensor data from an inertial measurement unit of the wearable device; electromyography (EMG) signals obtained using one or more EMG electrodes disposed in or on the wearable device; impedance plethysmography (IPG) data obtained using one or more sensors disposed in or on the wearable device; any combination thereof.

Light emitters of the one or more light emitters may be configured to emit light toward skin of a wearer of the wearable device using at least two different wavelengths of light.

At least one of the at least two different wavelengths of light may be in an infrared range.

PPG signals obtained using the at least two different wavelengths of light may be characterized to identify anatomical portions of the wearer involved in the at least one hand or finger gesture.

At least one light emitter and light detector pair of the one or more light emitters and the one or more light detectors may be spatially separated from another light emitter and light detector pair of the one or more light emitters and the one or more light detectors.

The wearable device may comprise a wrist-worn device.

In accordance with a second aspect, there is provided a wrist-worn wearable device comprising:
one or more light emitters and one or more light detectors, the one or more light emitters configured to emit light toward skin of a wearer of the wrist-worn wearable device and the one or more light detectors configured to capture light reflected from skin and/or internal body regions of the wearer;
a controller configured to:
   obtain a set of PPG signals, the set of PPG signals acquired using the one or more light emitters and the one or more light detectors disposed in or on a portion of a wrist-worn wearable device;
   separate the set of PPG signals into at least a physiological portion of the set of PPG signals and a motion artifact portion of the set of PPG signals; and
   identify at least one hand or finger gesture of the wearer of the wrist-worn wearable device based at least in part on the motion artifact portion of the set of PPG signals.

The wrist-worn wearable device may further comprise at least one of: an inertial measurement unit; at least one sensor configured to obtain impedance plethysmography signals; or one or more electromyography (EMG) electrodes, wherein the at least one hand or finger gesture is determined based on the inertial measurement unit, data obtained using the at least one sensor configured to obtain impedance plethysmography signals, or EMG signals obtained using the one or more EMG electrodes.

The wrist-worn wearable device may be communicatively coupled to an augmented reality (AR) or virtual reality (VR) headset, and wherein the at least one hand or finger gesture is used to control a functionality of the AR or VR headset.

At least one light emitter and light detector pair may be spatially separated from another light emitter and light detector pair.

The one or more light emitters and the one or more light detectors may be spatially separated and disposed along a back portion of a capsule of the wrist-worn wearable device.

Light emitters of the one or more light emitters may be configured to emit light toward skin of a wearer of the wrist-worn wearable device using at least two different wavelengths of light.

It will be appreciated that any features described herein as being suitable for incorporation into one or more aspects are intended to be generalizable across any and all aspects described herein. Other aspects can be understood by those skilled in the art in light of the description, the claims and the drawings. The foregoing general description and the following detailed description are exemplary and explanatory only and are not restrictive of the claims.

### BRIEF DESCRIPTION OF THE DRAWINGS

Illustrative embodiments are described in detail below with reference to the following figures.
FIG. 1A is a diagram that illustrates penetration depths of various wavelengths of light in accordance with some embodiments.
FIG. 1B is a diagram of portions of a hand and wrist region in accordance with some embodiments.
FIG. 1C illustrates example photoplethysmography data that includes motion artifact signals that may be used for hand and/or finger gesture detection in accordance with some embodiments.
FIG. 2 is a plan view of an example wristband system in accordance with some embodiments.
FIG. 3 is a schematic diagram of an exploded view of a portion of a wristband system that includes multiple light emitters and light detectors in accordance with some embodiments.
FIG. 4 is a flowchart of an example process for identifying a hand or finger gesture based on motion artifacts in a PPG signal in accordance with some embodiments.
FIG. 5 is a flowchart of an example process for identifying a hand or finger gesture based on a macromotion and a micromotion portion of a motion artifact of a PPG signal in accordance with some embodiments.
FIG. 6 is a simplified block diagram of an example of a computing system that may be implemented as part of a user device according to certain embodiments.
FIG. 7 is a simplified block diagram of an example of a computing system that may be implemented as part of a server according to certain embodiments.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION

Photoplethysmography (PPG) is used to determine physiological characteristics of a user. PPG may be implemented in wearable user devices, such as smart watches, smart glasses, AR/VR electronics, fitness trackers, etc. to determine physiological characteristics, such as heart rate, heart rate variability, oxygen saturation, blood pressure, and the like. However, PPG signals often have a motion artifact. For example, PPG signals obtained from a smart watch worn on a wrist of a user may have a motion artifact from, e.g., the user typing, walking, running, doing chores that involve motion of their hand/arm, etc. These motion artifacts may corrupt the PPG signals, making it more difficult to accurately determine the physiological characteristics of interest. Conventional techniques often involve removing the motion artifact from PPG signals prior to using the corrected PPG signals to determine various physiological characteristics.

Disclosed herein are techniques for utilizing the motion artifact of PPG signals to identify hand and/or finger gestures. The PPG signals may be obtained from a wrist-worn device, such as a smart watch or a fitness tracker. The PPG signals may be obtained from one or more light emitters and one or more light detectors disposed in or on a portion of the wrist-worn device. For example, such PPG signals may be used to characterize blood volume changes in blood vessels or tissue under the wearer's wrist. Motion artifacts present in these PPG signals may therefore characterize motion of various underlying wrist and/or hand anatomy, such as muscles that control the hand, the fingers, etc., and/or tendons that connect various muscles of the hand and wrist.

In some implementations, the PPG signals may be obtained using light emitters that emit light having multiple different wavelengths. Because each wavelength may penetrate to a different depth of tissue, PPG signals that span a variety of wavelengths may therefore capture information relating to a variety of depths of the wearer's body, spanning from surface level blood vessels (e.g., capillaries) to anatomical structures at relative depth (e.g., arteries, muscles, tendons, etc.). Additionally or alternatively, the PPG signals may be obtained using multiple light emitter and light detector pairs spanning different spatial location and emitter-detector distances, each pair corresponding to a different light path with potentially varying penetration depth and spatial location. By utilizing multiple light paths, the user's underlying anatomy may be probed and/or characterized across a spatial extent corresponding to all of the different light paths. In some implementations, the PPG signals may be multi-path and multi-wavelength, e.g., by utilizing both multiple light emitter and light detector pairs, and using multiple light emitters that emit light at different wavelengths, thereby allowing a larger volume of the body region of the user to be characterized, both spatially and depth-wise.

In some implementations, the motion artifact may be used to identify a hand or finger gesture, such as a motion of a particular finger (e.g., the index finger, the thumb, etc.) or a whole hand motion (e.g., opening the first, closing the fist, moving the whole hand laterally, flexing the wrist, etc.). The gesture may be identified by providing at least a portion of a motion artifact component of obtained PPG signals to a classification algorithm that generates an output indicative of the gesture. In some implementations, the motion artifact component may be identified by decomposing obtained PPG signals into a physiological portion and a motion artifact portion. As described herein, a "physiological portion" of a PPG signal generally refers to the portion of a PPG signal used to determine physiological characteristics, such as heart rate, heart rate variability, blood pressure, oxygen saturation, respiratory information, etc. The physiological portion of the PPG signal may include a cardiac components and respiratory components. The cardiac components may include information encoded in the PPG signal that contains information about the user's heart rate, heart rate variability, cardiac cycle (e.g., information indicative of fibrillation, tachycardia, bradycardia, electrocardiogram (ECG) signal, etc.), or the like. "Respiratory components" of a PPG signal refers to information encoded in the PPG signal that contains information about respiration including phase of breathing, rate of breathing, depth of breathing etc. In contrast, the "motion artifact portion" generally refers to artifact signals of a PPG signal that would conventionally be removed and/or discarded from the PPG signal prior to and/or as part of utilizing the PPG signal to determine physiological characteristics.

In some implementations, the hand and/or finger gesture may be utilized to control functionality of a different device, e.g., other than the wrist-worn wearable device from which the PPG signals were obtained. For example, in some embodiments, the gesture may be utilized to control functionality of a paired virtual reality (VR) or augmented reality (AR) headset or smart glasses. In some implementations, the wrist-worn wearable device may be communicatively coupled to the different device, e.g., via BLUETOOTH or other wireless communication channel such that an indication of the identified gesture may be transmitted to the other device for use.

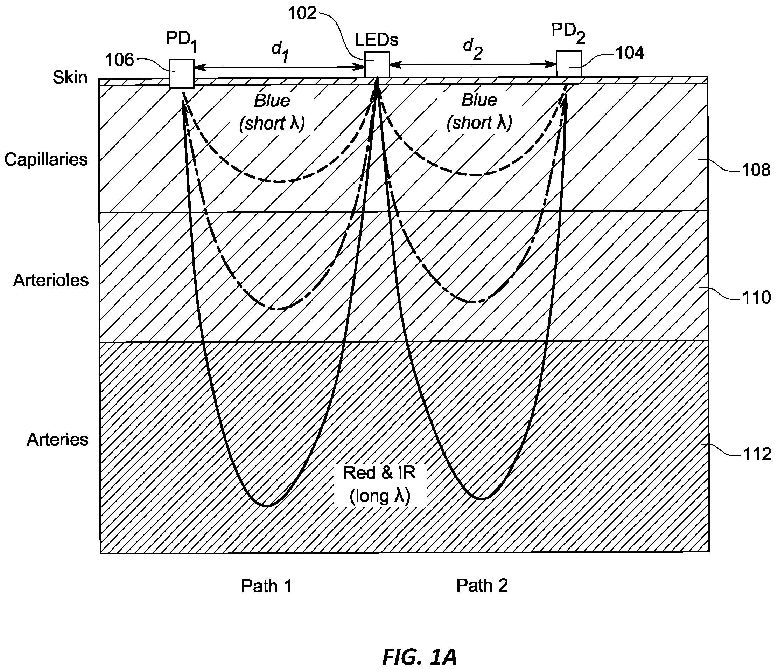

FIG. 1A is a diagram that illustrates varying penetration depths of different wavelengths of light in accordance with some embodiments. As illustrated, one or more light emitters, such as light emitting diode (LED) 102, and one or more light detectors, such as detectors 104 and 106, may be disposed proximate to a user's skin (e.g., proximate their finger ear or earlobe, forehead, toe, wrist, etc.). As illustrated, light may be emitted by LED 102 toward the skin of the user and may penetrate the skin to a given depth. The light may then reflect off the skin of the user and off various internal body regions (e.g., internal tissue, blood vessels, bone, muscles, tendons, etc.) and the reflected light may be captured by light detectors 104 and/or 106. Each light path from a light emitter to a light detector may be considered a path. Accordingly, in instances in which there are multiple possible light emitter and light detector pairs, the instance may be considered "multi-path," because reflected light (e.g., that originated from a given light emitter) may be captured by multiple possible light detectors, each corresponding to one path.

As illustrated, light emitted in relatively shorter wavelengths generally penetrates the body region at less depth compared to light in longer wavelengths. For example, as illustrated in FIG. 1A, blue wavelength light, which has a relatively short wavelength in the visible spectrum, may penetrate skin at a depth (depicted as depth 108) suitable for probing capillaries, which are surface level blood vessels. As another example, green wavelength light, which has a wavelength that is longer than blue light, may penetrate skin at a depth (depicted as depth 110) suitable for probing capillaries, and/or the vascular bed and arterioles, which are relatively small sized vessels for carrying arterial blood and are at a middle depth. As yet another example, red and/or infrared light, which are among the longest wavelengths used for PPG, may penetrate skin at the greatest depth (depicted as depth 112), which may be suitable for probing deeper blood vessels potentially arteries. Because red and infrared wavelength light probes the body region at the greatest depth, red and infrared wavelength light, when reflected off these body regions, may incorporate characteristics of relatively deep anatomical features other than arteries, such as tendons and/or muscles.

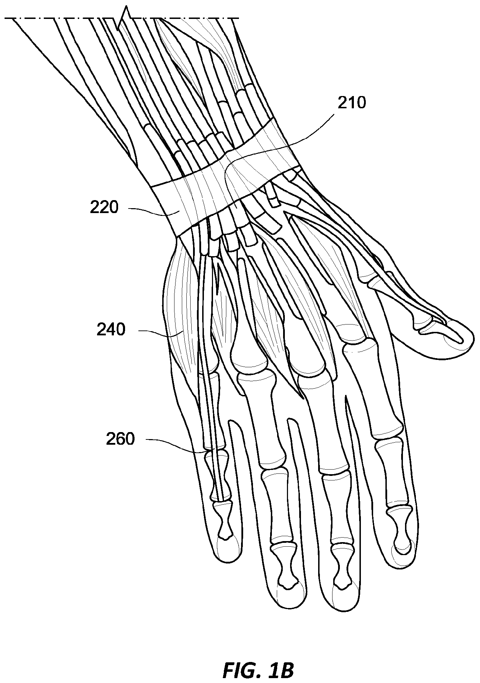

Because light of different wavelengths may penetrate the user's body to different depths, and because multiple light emitters and/or light detectors may be utilized to achieve multiple light paths spanning different spatial regions, a three-dimensional region of the user's body proximate the light emitters and/or light detectors may be essentially mapped. In other words, use of multiple light emitters and/or multiple light detectors may yield multiple light paths, which may span a given spatial location of the body region in two-dimensions. By using multiple light wavelengths, the body region may be characterized with respect to depth. In an instance in which PPG signals are obtained from light emitters and/or light detectors proximal a user's wrist, the body region may correspond to the user's wrist and underlying anatomy. Accordingly, reflected light may be used to characterize properties of underlying wrist, hand, and finger anatomy, such as the muscles and tendons of the wrist, hand, and fingers that generate movement of the wrist, hand, and fingers. FIG. 1B illustrates a diagram of anatomy of a hand, finger, and wrist region of a person. Various tendons and muscles that control movement of the fingers, hand, and wrist are illustrated in FIG. 1B, including tendon sheath of extensor digitorum 210, extensor retinaculum 220, abductor digiti minimi 240 and tendon of extensor digiti minimi 260. Movement of at least some of these muscles and tendons may be characterized by reflected light used to obtain PPG signals in accordance with some embodiments.

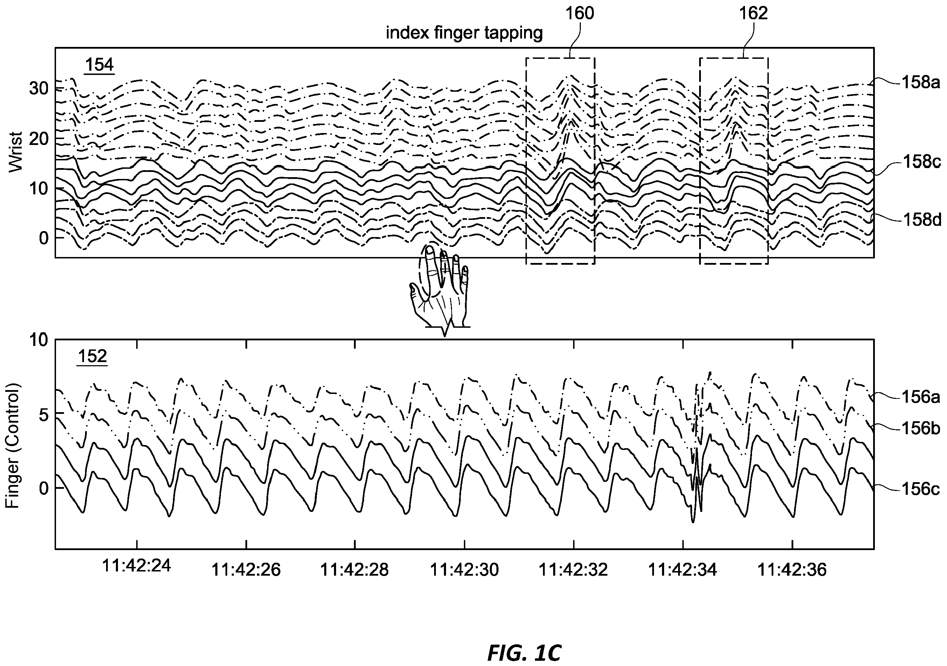

FIG. 1C illustrates experimental results of PPG data that include motion artifacts signals that may be used for finger and/or hand gesture detection in accordance with some embodiments. In the experimental data shown in FIG. 1C, PPG signals were obtained from a left hand fingertip of a user that remained still (e.g., motionless), as shown in panel 152. The PPG signals include signals from an infrared wavelength (depicted by curve 156a), signals from a red wavelength (depicted by 156b), and signals from a green wavelength (depicted by curve 156c). Panel 154 illustrates PPG signals obtained from a wrist of a right hand of the user, during a time period over which the index finger of the right hand was periodically tapping. Panel 154 includes PPG signals in an infrared wavelength (depicted by curve 158a), in a green wavelength (depicted by curve 158c), and in a blue wavelength (depicted by curve 158d). The PPG signals in panel 152 may be considered "ground truth" or "uncorrupted" PPG signals, as they were obtained when the user's hand and fingers from which the PPG signals were measured were at rest. The PPG signals in panel 154 include motion artifacts, e.g., as illustrated at time points 160 and 162. Note that the motion artifact is larger in amplitude for PPG signals obtained with relatively longer wavelength light, e.g., as shown by the motion artifacts visible in the PPG signals obtained in the infrared wavelength (e.g., in curve 158a).

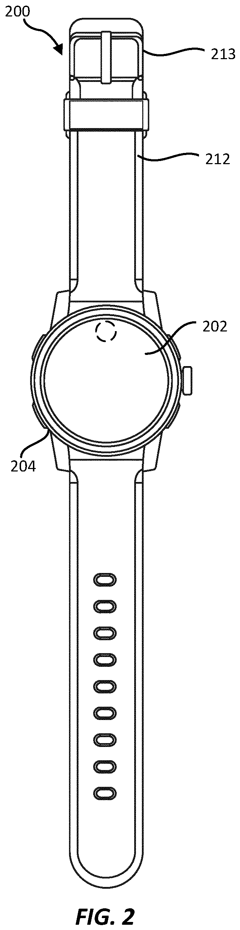

An example of a wearable user device that may include sensing electrodes is a wrist-worn device, such as a smart watch or fitness tracker. FIG. 2 illustrates an example wristband system 200 that includes a watch body 204 coupled to a watch band 212. Watch body 204 and watch band 212 may have any size and/or shape that is configured to allow a user to wear wristband system 200 on a body part (e.g., a wrist). Wristband system 200 may include a retaining mechanism 213 (e.g., a buckle) for securing watch band 212 to the user's wrist. Information, such as the time, date, measured user characteristics (e.g., physiological characteristics), etc. may be displayed on display 202. Display 202 may be a touchscreen such that the user may navigate through, e.g., menus, by touching portions of display 202. Wristband system 200 may perform various functions associated with the user. The functions may be executed independently in watch body 104, independently in watch band 212, and/or in communication between watch body 104 and watch band 212. Watch band 212 may be configured to operate independently (e.g., execute functions independently) from watch body 204. Additionally or alternatively, watch body 204 may be configured to operate independently (e.g., execute functions independently) from watch band 212. In some implementations, watch band 212 and/or watch body 204 may each include the independent resources required to independently execute functions. For example, watch band 212 and/or watch body 204 may each include a power source (e.g., a battery), a memory, data storage, a processor (e.g., a CPU), communications, a light source (e.g., at least one infrared LED for tracking watch body 204 and/or watch band 212 in space with an external sensor), and/or input/output devices. In some implementations, EMG electrodes for sensing EMG signals may be disposed in and/or on a portion of wristband system 200 that is configured to be in contact with the user's skin. For example, electrodes may be disposed on a back portion of watch band 212, a back portion of watch body 204, or any combination thereof.

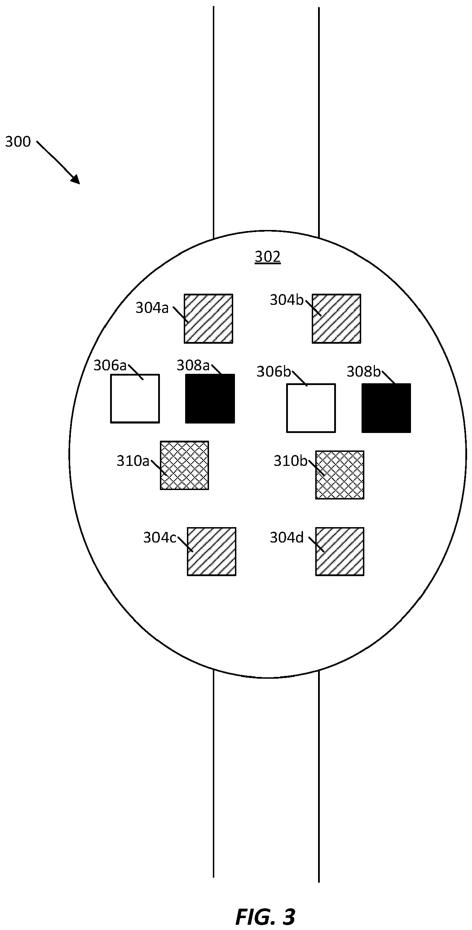

FIG. 3 is a diagram of an example wristband system that includes multiple light emitters and multiple light detectors for obtaining multi-wavelength, multi-path PPG signals in accordance with some embodiments. FIG. 3 illustrates a portion of a wrist-worn device 300 (e.g., a smart watch). Wrist-worn device 300 includes a capsule 302. Note that an exploded view of the back surface of capsule 302 is illustrated in FIG. 3. The back surface of capsule 302 is configured to rest on the wrist of the wearer of wrist-worn device 300. Disposed in or proximate to the back surface of capsule 302 are multiple light detectors, such as light detectors 304a, 304b, 304c, and 304d. Note that although four light detectors are illustrated in FIG. 3, other numbers, such as 1, 2, 3, 5, 8, etc. may be used. Each light detector may be, for example, a photodiode. Multiple light emitters are also disposed in or proximate to the back surface of capsule 302. For example, capsule 302 includes two light emitters 306a and 306b in a relatively short wavelength (e.g., in the blue wavelength), two light emitters 308a and 308b that emit light in a medium wavelength (e.g., in the green wavelength), and two light emitters 310a and 310b that emit light in a relatively long wavelength (e.g., in the red and/or infrared wavelength). Note that the relative positions of the light emitters and light detectors shown in FIG. 3, as well as the number of light emitters allocated to each wavelength, are merely exemplary, and other combinations may be used.

In some implementations, a hand or finger gesture of a wearer of a wearable device (e.g., a wearable wrist-worn device) may be determined based on a motion artifact of PPG signals obtained using the wearable device. For example, in some implementations, a set of PPG signals, which may be collected from multiple light emitter and light detector pairs and using multiple light wavelengths, may be separated into at least a physiological portion and a motion artifact portion. The physiological portion may be used for determining various physiological characteristics of the wearer, such as heart rate, heart rate variability, blood pressure, oxygen saturation, respiratory characteristics, etc. The motion artifact portion may be used to identify at least one hand or finger gesture. For example, the motion artifact portion may be provided to at least one machine learning algorithm that generates an indication of the hand or finger gesture as an output. For example, at least one machine learning algorithm may include a neural network, a classifier, or the like.

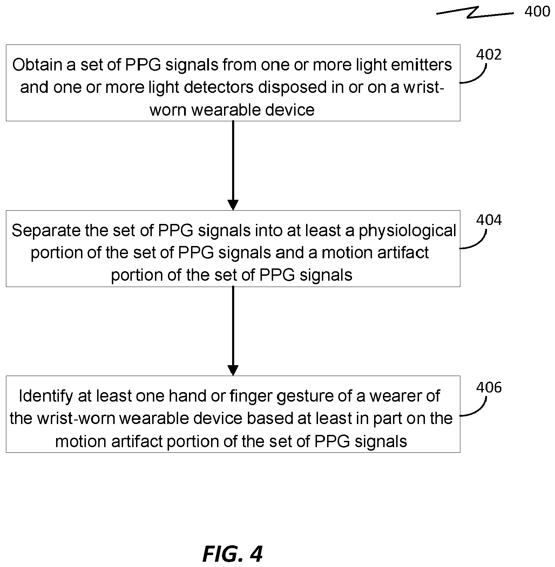

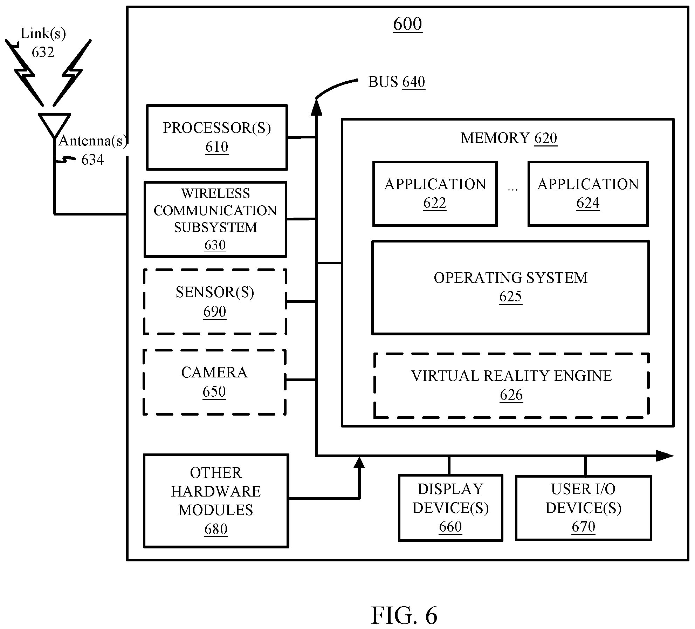

FIG. 4 is a flowchart of an example process 400 for identifying at least one hand or finger gesture based on a motion artifact portion of a set of PPG signals in accordance with some embodiments. In some embodiments, blocks of process 400 may be implemented by a processor or a controller of a wearable device. An example of a computing system that may be used in connection with such a wearable device is shown in and described below in connection with FIG. 6. In some embodiments, blocks of process 400 may be executed in an order other than what is shown in FIG. 4. In some implementations, two or more blocks of process 400 may be executed substantially in parallel. In some embodiments, one or more blocks of process 400 may be omitted.

Process 400 can begin at 402 by obtaining a set of PPG signals from one or more light emitters and one or more light detectors disposed in or on a wrist-worn wearable device. As shown in and described above in connection with FIG. 3, one or more light emitters may emit light at different wavelengths (e.g., a blue wavelength, a green wavelength, a red wavelength, an infrared wavelength, or any combination thereof). As described above in connection with FIGS. 1A and 1B, use of multiple wavelengths of emitted light may allow various depths of the body region of the wearer (e.g., the hand and wrist anatomy) to be probed due to the varying penetration depths of light at different wavelengths. The one or more light emitters and the one or more light detectors may be spatially separated from one other.

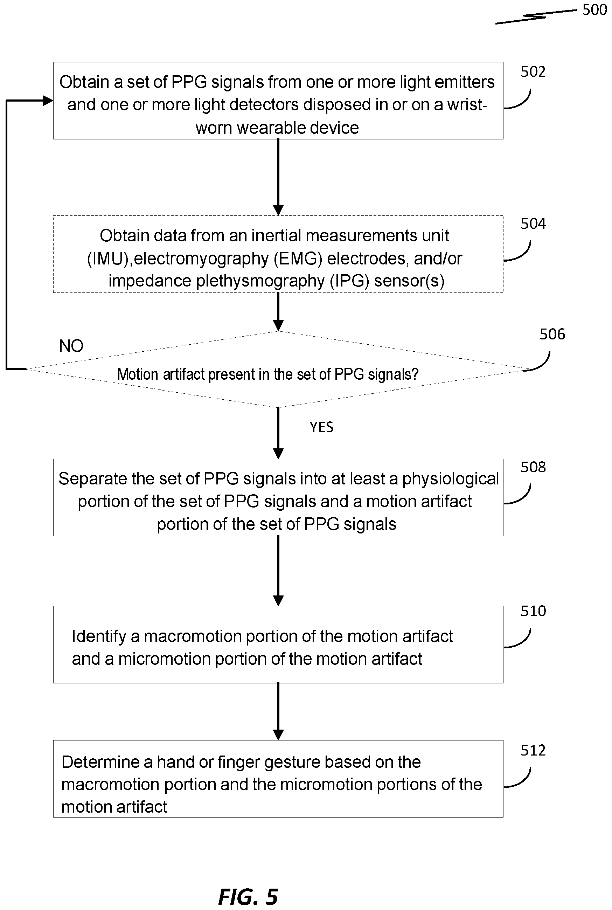

At 404, process 400 can separate the set of PPG signals into at least a physiological portion of the set of PPG signals and a motion artifact portion of the set of PPG signals. More detailed example techniques for separating the set of PPG signals into the physiological portion and the motion artifact portions are shown in and described below in connection with block 508 of FIG. 5. In some implementations, the physiological portion of the set of PPG signals may be used to determine various physiological characteristics of the wearer of the device, such as their heart rate, heart rate variability, blood pressure, oxygen saturation, respiratory characteristics, or the like.

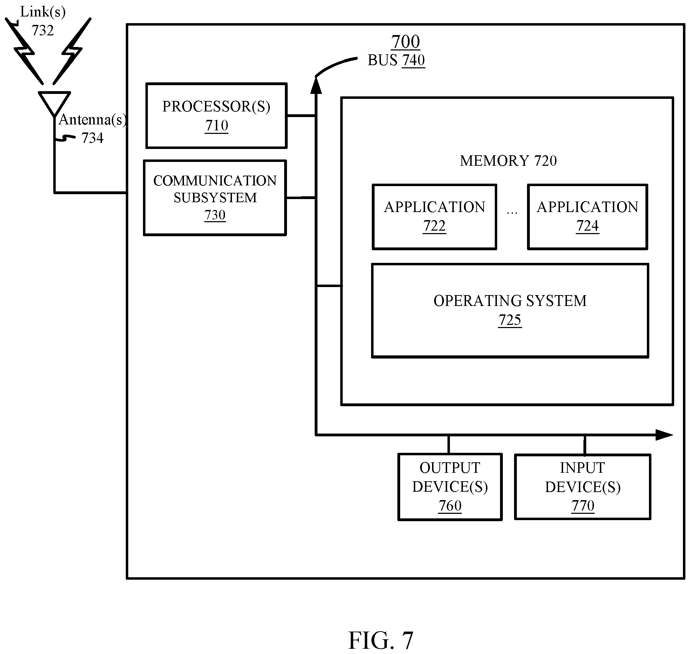

At 406, process 400 can identify at least one hand or finger gesture of a wearer of the wrist-worn wearable device based at least in part on the motion artifact portion of the set of PPG signals. Example gestures include: movement of a particular finger (e.g., the index finger, the thumb, the pinkie, etc.); a whole hand gesture (e.g., opening and/or closing the fist, flexion and/or extension of the wrist, lateral movement of the hand, etc.); movement of a subset of the fingers (e.g., a pinching motion of the index finger and the thumb, a snapping movement, etc.); or the like. For example, in some implementations, process 400 can provide the motion artifact portion of the set of PPG signals to a motion tracking system configured to output continuous tracking of motion or to a classification algorithm configured to output an indication of the gesture. The classification algorithm may be a trained machine learning algorithm, such as a trained neural network (e.g., a deep neural network, a convolutional neural network, etc.), a logistic regression, a support vector machine, a clustering algorithm, or the like. A co-adaption framework where both the motion tracking and gesture classification from PPG signal and user dexterity fine-tuning may also be used. In a co-adaptation framework, a feedback representation of user's performance in achieving certain motion pattern or gesture may be provided in real-time to the user, allowing both the user and the computer algorithm to co-adapt and converge to the optimal solution. In some embodiments, a co-adaptation framework may utilize a user interface that displays, in real-time or in near real-time, a representation of the user's performance in achieving certain motion patterns or gestures. Note that a model may be trained on a server device. An example of a computing system that may be used in connection with such a server device is shown in and described below in connection with FIG. 7.

In some implementations, after identifying at least one hand or finger gesture, a processor of the wearable device may cause an indication of the gesture to be transmitted to another device. For example, the other device may be a pair of smart glasses, an AR/VR headset, a media player (e.g., a smart television, a video game console system, etc.), a mobile device (e.g., a tablet computer, a mobile phone, etc.), or the like. The wrist-worn wearable device and the other device may be communicatively coupled, e.g., via a wireless communication channel, such as one that utilizes a BLUETOOTH protocol. The other device may then use the received gesture to control functionality of the other device. For example, the other device may identify an action to be performed (e.g., selecting a particular menu option, adjusting a volume of media playback, etc.) based on the received gesture, and may then cause that action to be performed.

FIG. 5 is a flowchart of an example process 500 for determining a hand or finger gesture based on macromotion and micromotion components of a motion artifact of a PPG signal in accordance with some embodiments. In some implementations, blocks of process 500 may be executed by a processor or a controller of a wrist-worn wearable device. In some embodiments, two or more blocks of process 500 may be executed substantially in parallel. In some embodiments, one or more blocks of process 500 may be omitted.

Process 500 can begin at 502 by obtaining a set of PPG signals from one or more light emitters and one or more light detectors disposed in or on a wrist-worn wearable device. Similar to what is described above in connection with blocks 402 of FIG. 4, as shown in and described above in connection with FIG. 3, the one or more light emitters may emit light at different wavelengths (e.g., a blue wavelength, a green wavelength, a red wavelength, an infrared wavelength, or any combination thereof). As described above in connection with FIGS. 1A and 1B, use of multiple wavelengths of emitted light may allow various depths of the body region of the wearer (e.g., the hand and wrist anatomy) to be probed due to the varying penetration depths of light at different wavelengths. The one or more light emitters and the one or more light detectors may be spatially separated from one other.

In some embodiments, at 504, process 500 can obtain data from an inertial measurement unit (IMU), one or more electromyography (EMG) electrodes, and/or one or more impedance plethysmography (IPG) sensors. The IMU, EMG electrodes, and/or the IPG sensors may be disposed in or on any suitable portion of the wrist-worn device. For example, the IMU may be disposed within a capsule of the wrist-worn device. As another example, the EMG electrodes may be disposed in or on a backside portion of the capsule of the wrist-worn device and/or along a band of the wrist-worn device such that the EMG electrodes are configured to be in contact with skin of the wearer. Note that, in some embodiments, process 500 can obtain data from other sensors or components, such as a camera sensor of the wrist-worn device. In instances in which block 504 is omitted, process 500 can proceed directly to block 506.

In some embodiments, at 506, process 500 can determine whether there is motion artifact present in the set of PPG signals. In some implementations, process 500 may determine whether there is motion artifact present in the set of PPG signals based on any combination of the IMU data, EMG signals, and/or IPG data. For example, process 500 may determine there is motion artifact present in the set of PPG signals based on the IMU data and/or the EMG signals having an amplitude greater than a predetermined or adaptive threshold. In some implementations, process 500 may determine whether there is motion artifact present in the set of PPG signals by comparing the set of PPG signals to a ground truth or canonical set of PPG signals that represent optimal PPG signals (e.g., without any motion artifact). For example, in some embodiments, process 500 can determine a correlation between the set of PPG signals and the canonical set of PPG signals and can determine that there is motion artifact present in the set of PPG signals if the correlation is less than a predetermined or adaptive threshold.

If, at block 506, process 500 determines that there is no motion artifact present in the set of PPG signals ("no" at 506), process 500 can loop back to block 502 and can obtain additional PPG signals.

Conversely, if at block 506, process 500 determines that there is motion artifact present in the set of PPG signals ("yes" at 506), process 500 can proceed to block 508 and can separate the set of PPG signals into at least a physiological portion and a motion artifact portion. Note that, in instances in which block 506 is omitted, process 500 can proceed directly to block 508. In some implementations, process 500 can separate the set of PPG signals into the physiological portion and the motion artifact portion by utilizing an adaptive filter. For example, the adaptive filter may utilize a PPG signal from a relatively longer wavelength (e.g., in the green wavelength) to identify an error corresponding to the motion artifact in a PPG signal of a shorter wavelength (e.g., in the red or infrared wavelength). Other example techniques that may be utilized to separate the set of PPG signals into the physiological portion and the motion artifact portion include: an adaptive spectral subtraction method, a time-domain adaptive filter, principal component analysis (PCA), singular spectral decomposition, independent component analysis (ICA), cross-correlation and/or a cross-coherence approach, or any combination thereof. Note that, in some embodiments, process 500 can utilize data from PPG signals and/or non-PPG signals (e.g., data obtained at block 504) to separate the PPG signals into the physiological portion and the motion artifact portion. For example, process 500 may utilize any of camera data, IPG data, IMU data, EMG data, or the like. As a more particular example, in instances in which a trained machine learning or signal separation model is used to separate the physiological portion and the motion artifact portion, camera data, IPG data, IMU data, and/or EMG data may be provided in conjunction with the PPG signals to the trained model, which may be configured to generate, as an output, a "cleaned" version of the PPG signals corresponding to the physiological portion. A difference between the cleaned version and the original PPG signals may be considered the motion artifact portion of the PPG signals. In some embodiments, such a trained model may be trained on a data set that includes PPG signals obtained without any motion (e.g., obtained with a user being still), PPG signals with motion artifact (e.g., with the user making various hand/finger motions), and other data obtained during the time period the user was moving during with this PPG signals with motion artifact was obtained (e.g., the IMU data, the IPG data, the EMG data, the camera data, etc. collected or obtained during that time period). In some embodiments, a signal separation model configured to separate a PPG signal into a physiological portion and a motion artifact portion may be based on the model of heart rate kinetics. Such a model of heart rate kinetics may utilize an underlying model for a cardiac cycle, e.g., toutilize known properties of the heart, such as its periodicity and other kinetics. Any suitable version of such a model may be trained to predict clean PPG signals (e.g., comprising only the physiological portion), and the residual signal can be used in tandem with other modalities for gesture recognition.

At 510, process 500 can identify a macromotion portion and a micromotion portion of the motion artifact. In some implementations, the macromotion portion may correspond to whole-hand or multiple finger movements of the hand and/or wrist. For example, the macromotion portion may indicate hand gestures such as opening and/or closing the first, waving the whole hand, flexion or extension of the wrist, two fingers being pinched together, the hand being shaped into a given shape (e.g., a shape associated with sign language), etc. In contrast, the micromotion portion may represent movement of individual fingers, such as a finger tapping, a finger being bent at a knuckle, etc. In addition, the micromotion portion may represent modification of the tension of the muscles or tendons in the sensor area without externally visible movement.

In some implementations, the macromotion and micromotion portions may be determined by measuring a strength of a dominance of the periodicity in the set of PPG signals for a given wavelength by taking a ratio of energy in the peak frequency and subsequent harmonics to the energy in remainder of frequency band of the signals. This metric is generally referred to herein as the pSNR, and PPG signals exhibiting a random amount of micromotion may exhibit a relatively low pSNR, whereas PPG signals exhibiting either no micromotion or a periodic micromotion may exhibit a relatively high pSNR. Alternative metrics such as Crest Factor (CF) may also be used.

In some implementations, the macromotion and micromotion portions may be determined by determining a perfusion index of PPG signals of a relatively low wavelength (e.g., green) to the perfusion index of PPG signals of a higher wavelength (e.g., infrared). The ratio of perfusion indices may exhibit a relatively high value when there is no micromotion present, and a lower value when there is either periodic or random micromotion present.

In some implementations, the macromotion and micromotion portions may be determined by determining a skewness of the PPG signals for a given wavelength. In instances in which there is no micromotion, the skewness metric may be within a given predetermined range, whereas the skewness metric may be outside the predetermined range when there is either periodic or random micromotion.

In some implementations, the macromotion and micromotion portions may be determined by determining a maximum correlation to a waveform bank that includes canonical PPG waveforms without motion artifact and/or without micromotion artifacts. The correlation may be high in instances in which there is no micromotion, and low in instances when there is either periodic or random micromotion.

At 512, process 500 can determine a hand or finger gesture based on the macromotion portion and the micromotion portion of the motion artifact. For example, in some implementations, process 500 can provide the macromotion portion and/or the micromotion portion to a trained machine learning algorithm trained to output the hand or finger gesture as a classification. Examples of such machine learning algorithms include clustering algorithms, a deep learning network, a recurrent neural network, a long short-term memory network, a convolutional neural network, or the like. As another example, in some implementations, process 500 may use a distance-based metric to compute distances between a representation of the micromotion portion and/or the macromotion portion to signatures of known hand/finger gestures. The distance-based metric may include a Euclidean distance, a Mahalanobis distance, or the like. As yet another example, in some implementations, process 500 may use a dictionary-based approach that identifies a signature of a known hand or finger gesture that corresponds to the macromotion and/or the micromotion portions of the motion artifact. A dictionary-based approach may be implemented with dynamic time warping (DTW). In some embodiments, a model ensemble may be used that incorporates multiple different models (which may have different model types) and identifies a gesture that corresponds to a consensus of the multiple different models.

**FIG. 6** is a simplified block diagram of an example of a computing system 600 for implementing some of the examples described herein. For example, computing system 600 may represent a computing system associated with a wrist-worn device, and one or more processors of computing system 600 may perform any of the techniques described above in connection with FIGS. 4 and/or 5. In the illustrated example, computing system 600 may include one or more processor(s) 610 and a memory 620. Processor(s) 610 may be configured to execute instructions for performing operations at a number of components, and can be, for example, a general-purpose processor or microprocessor suitable for implementation within a portable electronic device, a wearable device (e.g., a smart watch, a fitness tracker, an AR/VR headset or controller, etc.), a mobile device (e.g., a mobile phone, a tablet computer, etc.). Processor(s) 610 may be communicatively coupled with a plurality of components within computing system 600. To realize this communicative coupling, processor(s) 610 may communicate with the other illustrated components across a bus 640. Bus 640 may be any subsystem adapted to transfer data within computing system 600. Bus 640 may include a plurality of computer buses and additional circuitry to transfer data.

Memory 620 may be coupled to processor(s) 610. In some embodiments, memory 620 may offer both short-term and long-term storage and may be divided into several units. Memory 620 may be volatile, such as static random access memory (SRAM) and/or dynamic random access memory (DRAM) and/or non-volatile, such as read-only memory (ROM), flash memory, and the like. Furthermore, memory 620 may include removable storage devices, such as secure digital (SD) cards. Memory 620 may provide storage of computer-readable instructions, data structures, program modules, and other data for computing system 600. In some embodiments, memory 620 may be distributed into different hardware modules. A set of instructions and/or code might be stored on memory 620. The instructions might take the form of executable code that may be executable by computing system 600, and/or might take the form of source and/or installable code, which, upon compilation and/or installation on computing system 600 (e.g., using any of a variety of generally available compilers, installation programs, compression/decompression utilities, etc.), may take the form of executable code.

In some embodiments, memory 620 may store a plurality of application modules 622 through 624, which may include any number of applications. Examples of applications may include gaming applications, conferencing applications, video playback applications, or other suitable applications. The applications may include a depth sensing function or eye tracking function. Application modules 622- 624 may include particular instructions to be executed by processor(s) 610. In some embodiments, certain applications or parts of application modules 622- 624 may be executable by other hardware modules 680. In certain embodiments, memory 620 may additionally include secure memory, which may include additional security controls to prevent copying or other unauthorized access to secure information.

In some embodiments, memory 620 may include an operating system 625 loaded therein. Operating system 625 may be operable to initiate the execution of the instructions provided by application modules 622- 624 and/or manage other hardware modules 680 as well as interfaces with a wireless communication subsystem 630 which may include one or more wireless transceivers. Operating system 625 may be adapted to perform other operations across the components of computing system 600 including threading, resource management, data storage control and other similar functionality.

Wireless communication subsystem 630 may include, for example, an infrared communication device, a wireless communication device and/or chipset (such as a Bluetooth^{®} device, an IEEE 802.11 device, a Wi-Fi device, a WiMax device, cellular communication facilities, etc.), and/or similar communication interfaces. Computing system 600 may include one or more antennas 634 for wireless communication as part of wireless communication subsystem 630 or as a separate component coupled to any portion of the system. Depending on desired functionality, wireless communication subsystem 630 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.17) network. A WLAN may be, for example, an IEEE 802.11x network. A WPAN may be, for example, a Bluetooth network, an IEEE 802.7x, or some other types of network. The techniques described herein may also be used for any combination of WWAN, WLAN, and/or WPAN. Wireless communications subsystem 630 may permit data to be exchanged with a network, other computer systems, and/or any other devices described herein. Wireless communication subsystem 630 may include a means for transmitting or receiving data, such as identifiers of HMD devices, position data, a geographic map, a heat map, photos, or videos, using antenna(s) 634 and wireless link(s) 632. Wireless communication subsystem 630, processor(s) 610, and memory 620 may together comprise at least a part of one or more of a means for performing some functions disclosed herein.

Embodiments of computing system 600 may also include one or more sensors 690. Sensor(s) 690 may include, for example, an image sensor, an accelerometer, a force sensor, a hydrostatic pressure sensor, a temperature sensor, a proximity sensor, a magnetometer, a gyroscope, an inertial sensor (e.g., a module that combines an accelerometer and a gyroscope), an ambient light sensor, or any other similar module operable to provide sensory output and/or receive sensory input, such as a depth sensor or a position sensor. For example, in some implementations, sensor(s) 690 may include one or more inertial measurement units (IMUs) and/or one or more position sensors. An IMU may generate calibration data indicating an estimated position of a device, based on measurement signals received from one or more of the position sensors. A position sensor may generate one or more measurement signals in response to motion of a device. Examples of the position sensors may include, but are not limited to, one or more accelerometers, one or more gyroscopes, one or more magnetometers, another suitable type of sensor that detects motion, a type of sensor used for error correction of the IMU, or some combination thereof. The position sensors may be located external to the IMU, internal to the IMU, or some combination thereof. At least some sensors may use a structured light pattern for sensing.

Computing system 600 may include a display module 660. Display module 660 may be a near-eye display, and may graphically present information, such as images, videos, and various instructions, from computing system 600 to a user. Such information may be derived from one or more application modules 622- 624, virtual reality engine 626, one or more other hardware modules 680, a combination thereof, or any other suitable means for resolving graphical content for the user (e.g., by operating system 625). Display module 660 may use liquid crystal display (LCD) technology, light-emitting diode (LED) technology (including, for example, OLED, ILED, □LED, AMOLED, TOLED, etc.), light emitting polymer display (LPD) technology, or some other display technology.

Computing system 600 may include a user input/output module 670. User input/output module 670 may allow a user to send action requests to computing system 600. An action request may be a request to perform a particular action. For example, an action request may be to start or end an application or to perform a particular action within the application. User input/output module 670 may include one or more input devices. Example input devices may include a touchscreen, a touch pad, microphone(s), button(s), dial(s), switch(es), a keyboard, a mouse, a game controller, or any other suitable device for receiving action requests and communicating the received action requests to computing system 600. In some embodiments, user input/output module 670 may provide haptic feedback to the user in accordance with instructions received from computing system 600. For example, the haptic feedback may be provided when an action request is received or has been performed.

Computing system 600 may include a camera 650 that may be used to take photos or videos. Camera 650 may be configured to take photos or videos of the user. Camera 650 may also be used to take photos or videos of the environment, for example, for VR, AR, or MR applications. Camera 650 may include, for example, a complementary metal-oxide-semiconductor (CMOS) image sensor with a few millions or tens of millions of pixels. In some implementations, camera 650 may include two or more cameras that may be used to capture 3-D images.

In some embodiments, computing system 600 may include a plurality of other hardware modules 680. Each of other hardware modules 680 may be a physical module within computing system 600. While each of other hardware modules 680 may be permanently configured as a structure, some of other hardware modules 680 may be temporarily configured to perform specific functions or temporarily activated. Examples of other hardware modules 680 may include, for example, an audio output and/or input module (e.g., a microphone or speaker), a near field communication (NFC) module, a rechargeable battery, a battery management system, a wired/wireless battery charging system, etc. In some embodiments, one or more functions of other hardware modules 680 may be implemented in software.

In some embodiments, memory 620 of computing system 600 may also store a virtual reality engine 626. Virtual reality engine 626 may execute applications within computing system 600 and receive position information, acceleration information, velocity information, predicted future positions, or some combination thereof from the various sensors. In some embodiments, the information received by virtual reality engine 626 may be used for producing a signal (e.g., display instructions) to display module 660. For example, if the received information indicates that the user has looked to the left, virtual reality engine 626 may generate content that mirrors the user's movement in a virtual environment. Additionally, virtual reality engine 626 may perform an action within an application in response to an action request received from user input/output module 570 and provide feedback to the user. The provided feedback may be visual, audible, or haptic feedback. In some implementations, processor(s) 610 may include one or more GPUs that may execute virtual reality engine 526.

In various implementations, the above-described hardware and modules may be implemented on a single device or on multiple devices that can communicate with one another using wired or wireless connections. For example, in some implementations, some components or modules, such as GPUs, virtual reality engine 626, and applications (e.g., tracking application), may be implemented on two or more paired or connected devices.

In alternative configurations, different and/or additional components may be included in computing system 600. Similarly, functionality of one or more of the components can be distributed among the components in a manner different from the manner described above. For example, in some embodiments, computing system 600 may be modified to include other system environments, such as an AR system environment and/or an MR environment.

**FIG. 7** is a simplified block diagram of an example of a computing system 700 that may be implemented in connection with a server in accordance with some embodiments. For example, computing system 700 may be used to implement a server used to generate and/or train various machine learning models, or the like. For example, a machine learning model may be trained to identify and/or detect particular gestures based on motion artifact data.

In the illustrated example, computing system 700 may include one or more processor(s) 710 and a memory 720. Processor(s) 710 may be configured to execute instructions for performing operations at a number of components, and can be, for example, a general-purpose processor or microprocessor suitable for implementation within a server device. Processor(s) 710 may be communicatively coupled with a plurality of components within computing system 700. To realize this communicative coupling, processor(s) 710 may communicate with the other illustrated components across a bus 740. Bus 740 may be any subsystem adapted to transfer data within computing system 700. Bus 740 may include a plurality of computer buses and additional circuitry to transfer data.

Memory 720 may be coupled to processor(s) 710. In some embodiments, memory 720 may offer both short-term and long-term storage and may be divided into several units. Memory 720 may be volatile, such as static random access memory (SRAM) and/or dynamic random access memory (DRAM) and/or non-volatile, such as read-only memory (ROM), flash memory, and the like. Furthermore, memory 720 may include removable storage devices, such as secure digital (SD) cards. Memory 720 may provide storage of computer-readable instructions, data structures, program modules, and other data for computing system 700. In some embodiments, memory 720 may be distributed into different hardware modules. A set of instructions and/or code might be stored on memory 720. The instructions might take the form of executable code that may be executable by computing system 700, and/or might take the form of source and/or installable code, which, upon compilation and/or installation on computing system 700 (e.g., using any of a variety of generally available compilers, installation programs, compression/decompression utilities, etc.), may take the form of executable code.

In some embodiments, memory 720 may store a plurality of application modules 722 through 724, which may include any number of applications. Examples of applications may include gaming applications, conferencing applications, video playback applications, or other suitable applications. Application modules 722- 724 may include particular instructions to be executed by processor(s) 710. In some embodiments, certain applications or parts of application modules 722-724 may be executable by other hardware modules. In certain embodiments, memory 720 may additionally include secure memory, which may include additional security controls to prevent copying or other unauthorized access to secure information.

In some embodiments, memory 720 may include an operating system 725 loaded therein. Operating system 725 may be operable to initiate the execution of the instructions provided by application modules 722- 724 and/or manage other hardware modules as well as interfaces with a wireless communication subsystem 730 which may include one or more wireless transceivers. Operating system 725 may be adapted to perform other operations across the components of computing system 700 including threading, resource management, data storage control and other similar functionality.

Communication subsystem 730 may include, for example, an infrared communication device, a wireless communication device and/or chipset (such as a Bluetooth^{®} device, an IEEE 802.11 device, a Wi-Fi device, a WiMax device, cellular communication facilities, etc.), a wired communication interface, and/or similar communication interfaces. Computing system 700 may include one or more antennas 734 for wireless communication as part of wireless communication subsystem 730 or as a separate component coupled to any portion of the system. Depending on desired functionality, communication subsystem 730 may include separate transceivers to communicate with base transceiver stations and other wireless devices and access points, which may include communicating with different data networks and/or network types, such as wireless wide-area networks (WWANs), wireless local area networks (WLANs), or wireless personal area networks (WPANs). A WWAN may be, for example, a WiMax (IEEE 802.17) network. A WLAN may be, for example, an IEEE 802.11x network. A WPAN may be, for example, a Bluetooth network, an IEEE 802.8x, or some other types of network. The techniques described herein may also be used for any combination of WWAN, WLAN, and/or WPAN. Communications subsystem 730 may permit data to be exchanged with a network, other computer systems, and/or any other devices described herein. Communication subsystem 730 may include a means for transmitting or receiving data, using antenna(s) 734, wireless link(s) 732, or a wired link. Communication subsystem 730, processor(s) 710, and memory 720 may together comprise at least a part of one or more of a means for performing some functions disclosed herein.

In some embodiments, computing system 700 may include one or more output device(s) 760 and/or one or more input device(s) 770. Output device(s) 770 and/or input device(s) 770 may be used to provide output information and/or receive input information.

Embodiments disclosed herein may be used to implement components of an artificial reality system or may be implemented in conjunction with an artificial reality system. Artificial reality is a form of reality that has been adjusted in some manner before presentation to a user, which may include, for example, a virtual reality, an augmented reality, a mixed reality, a hybrid reality, or some combination and/or derivatives thereof. Artificial reality content may include completely generated content or generated content combined with captured (e.g., real-world) content. The artificial reality content may include video, audio, haptic feedback, or some combination thereof, and any of which may be presented in a single channel or in multiple channels (such as stereo video that produces a three-dimensional effect to the viewer). Additionally, in some embodiments, artificial reality may also be associated with applications, products, accessories, services, or some combination thereof, that are used to, for example, create content in an artificial reality and/or are otherwise used in (e.g., perform activities in) an artificial reality. The artificial reality system that provides the artificial reality content may be implemented on various platforms, including an HMD connected to a host computer system, a standalone HMD, a mobile device or computing system, or any other hardware platform capable of providing artificial reality content to one or more viewers.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the scope of the appended claims.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

With reference to the appended figures, components that can include memory can include non-transitory machine-readable media. The term "machine-readable medium" and "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean any combination of A, B, and/or C, such as A, AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

### Example embodiments:

Embodiment 1: A method for utilizing motion artifacts in photoplethysmography (PPG) signals, the method comprising: obtaining a set of PPG signals, the set of PPG signals acquired using one or more light emitters and one or more light detectors disposed in or on a portion of a wearable device; separating the set of PPG signals into at least a physiological portion of the set of PPG signals and a motion artifact portion of the set of PPG signals; and identifying at least one hand or finger gesture of a wearer of the wearable device based at least in part on the motion artifact portion of the set of PPG signals.

Embodiment 2: The method of embodiment 1, wherein the wearable device is a wrist-worn device.

Embodiment 3: The method of any one of embodiments 1 or 2, wherein at least one light emitter and light detector pair of the one or more light emitters and the one or more light detectors is spatially separated from another light emitter and light detector pair of the one or more light emitters and the one or more light detectors.

Embodiment 4: The method of any one of embodiments 1-3, wherein light emitters of the one or more light emitters are configured to emit light toward skin of a wearer of the wearable device using at least two different wavelengths of light.

Embodiment 5: The method of any one of embodiments 3 or 4, wherein at least one of the at least two different wavelengths of light is in an infrared range.

Embodiment 6: The method of any one of embodiments 3-5, wherein PPG signals obtained using the at least two different wavelengths of light are characterized to identify anatomical portions of the wearer involved in the at least one hand or finger gesture.

Embodiment 7: The method of any one of embodiments 1-6, wherein the at least one hand or finger gesture comprises at least one of: motion of an individual finger; motion of two or more fingers; a fist-closing gesture; a hand-waving gesture; or any combination thereof.

Embodiment 8: The method of any one of embodiments 1-7, wherein the at least one hand or finger gesture is used to control functionality of an augmented reality (AR) or virtual reality (VR) headset paired with the wearable device.

Embodiment 9: The method of any one of embodiments 1-8, further comprising, prior to separating the set of PPG signals into at least the physiological portion of the set of PPG signals and the motion artifact portion of the set of PPG signals, determining that the set of PPG signals includes the motion artifacts.

Embodiment 10: The method of embodiment 9, wherein determining that the set of PPG signals includes the motion artifacts is based on at least one of: a perfusion index at two different wavelengths of light of the set of PPG signals; a periodicity in the set of PPG signals; a skewness of the set of PPG signals; correlation of the set of PPG signals to a ground truth PPG signal without motion artifact; or any combination thereof.

Embodiment 11: The method of any one of embodiments 1-10, wherein separating the set of PPG signals into at least the physiological portion of the set of PPG signals and the motion artifact portion of the set of PPG signals comprises using an adaptive filter.

Embodiment 12: The method of any one of embodiments 1-11, further comprising separating the motion artifact portion of the set of PPG signals into a macromotion portion representing motion of multiple fingers and a micromotion portion representing motion of individual fingers.

Embodiment 13: The method of any one of embodiments 1-12, wherein identifying the at least one hand or finger gesture comprises applying a trained classifier to at least the motion artifact portion of the set of PPG signals.

Embodiment 14: The method of any one of embodiments 1-13, wherein identifying the at least one hand or finger gesture comprises utilizing at least one of: sensor data from an inertial measurement unit of the wearable device; electromyography (EMG) signals obtained using one or more EMG electrodes disposed in or on the wearable device; impedance plethysmography (IPG) data obtained using one or more sensors disposed in or on the wearable device; any combination thereof.

Embodiment 15: A wrist-worn wearable device, comprising: one or more light emitters and one or more light detectors, the one or more light emitters configured to emit light toward skin of a wearer of the wrist-worn wearable device and the one or more light detectors configured to capture light reflected from skin and/or internal body regions of the wearer; and a controller configured to: obtain a set of PPG signals, the set of PPG signals acquired using the one or more light emitters and the one or more light detectors disposed in or on a portion of a wrist-worn wearable device, separate the set of PPG signals into at least a physiological portion of the set of PPG signals and a motion artifact portion of the set of PPG signals, and identify at least one hand or finger gesture of the wearer of the wrist-worn wearable device based at least in part on the motion artifact portion of the set of PPG signals.

Embodiment 16: The wrist-worn wearable device of embodiment 15, further comprising at least one of: an inertial measurement unit; at least one sensor configured to obtain impedance plethysmography signals; or one or more electromyography (EMG) electrodes, wherein the at least one hand or finger gesture is determined based on the inertial measurement unit, data obtained using the at least one sensor configured to obtain impedance plethysmography signals, or EMG signals obtained using the one or more EMG electrodes.

Embodiment 17: The wrist-worn wearable device of any one of embodiments 15 or 16, wherein the wrist-worn wearable device is communicatively coupled to an augmented reality (AR) or virtual reality (VR) headset, and wherein the at least one hand or finger gesture is used to control a functionality of the AR or VR headset.

Embodiment 18: The wrist-worn wearable device of any one of embodiments 15-17, wherein at least one light emitter and light detector pair is spatially separated from another light emitter and light detector pair.

Embodiment 19: The wrist-worn wearable device of any one of embodiments 15-18, wherein the one or more light emitters and the one or more light detectors are spatially separated and disposed along a back portion of a capsule of the wrist-worn wearable device.

Embodiment 20: The wrist-worn wearable device of any one of embodiments 15-19, wherein light emitters of the one or more light emitters are configured to emit light toward skin of a wearer of the wearable device using at least two different wavelengths of light.

The specification and drawings are, accordingly, to be regarded in an illustrative rather than a restrictive sense. It will, however, be evident that additions, subtractions, deletions, and other modifications and changes may be made thereunto within the scope of the appended claims. Thus, although specific embodiments have been described, these are not intended to be limiting. Various modifications and equivalents are within the scope of the following claims.

## Claims

1. A method for utilizing motion artifacts in photoplethysmography (PPG) signals, the method comprising:
obtaining a set of PPG signals, the set of PPG signals acquired using one or more light emitters and one or more light detectors disposed in or on a portion of a wearable device;
separating the set of PPG signals into at least a physiological portion of the set of PPG signals and a motion artifact portion of the set of PPG signals; and
identifying at least one hand or finger gesture of a wearer of the wearable device based at least in part on the motion artifact portion of the set of PPG signals.

2. The method of claim 1, and one or more of the following:
(i) wherein the at least one hand or finger gesture comprises at least one of: motion of an individual finger; motion of two or more fingers; a fist-closing gesture; a hand-waving gesture; or any combination thereof;
(ii) wherein the at least one hand or finger gesture is used to control functionality of an augmented reality (AR) or virtual reality (VR) headset paired with the wearable device.

3. The method of claim 1 or claim 2, further comprising, prior to separating the set of PPG signals into at least the physiological portion of the set of PPG signals and the motion artifact portion of the set of PPG signals, determining that the set of PPG signals includes the motion artifacts;
optionally, wherein determining that the set of PPG signals includes the motion artifacts is based on at least one of: a perfusion index at two different wavelengths of light of the set of PPG signals; a periodicity in the set of PPG signals; a skewness of the set of PPG signals; correlation of the set of PPG signals to a ground truth PPG signal without motion artifact; or any combination thereof.

4. The method of any preceding claim, wherein separating the set of PPG signals into at least the physiological portion of the set of PPG signals and the motion artifact portion of the set of PPG signals comprises using an adaptive filter.

5. The method of any preceding claim, further comprising separating the motion artifact portion of the set of PPG signals into a macromotion portion representing motion of multiple fingers and a micromotion portion representing motion of individual fingers.

6. The method of any preceding claim, wherein identifying the at least one hand or finger gesture comprises applying a trained classifier to at least the motion artifact portion of the set of PPG signals.

7. The method of any preceding claim, wherein identifying the at least one hand or finger gesture comprises utilizing at least one of: sensor data from an inertial measurement unit of the wearable device; electromyography (EMG) signals obtained using one or more EMG electrodes disposed in or on the wearable device; impedance plethysmography (IPG) data obtained using one or more sensors disposed in or on the wearable device; any combination thereof.

8. The method of any preceding claim, wherein light emitters of the one or more light emitters are configured to emit light toward skin of a wearer of the wearable device using at least two different wavelengths of light;
optionally, wherein at least one of the at least two different wavelengths of light is in an infrared range;
optionally, wherein PPG signals obtained using the at least two different wavelengths of light are characterized to identify anatomical portions of the wearer involved in the at least one hand or finger gesture.

9. The method of any preceding claim, wherein at least one light emitter and light detector pair of the one or more light emitters and the one or more light detectors is spatially separated from another light emitter and light detector pair of the one or more light emitters and the one or more light detectors.

10. The method of any preceding claim, wherein the wearable device is a wrist-worn device.

11. A wrist-worn wearable device, comprising:
one or more light emitters and one or more light detectors, the one or more light emitters configured to emit light toward skin of a wearer of the wrist-worn wearable device and the one or more light detectors configured to capture light reflected from skin and/or internal body regions of the wearer; and
a controller configured to:
obtain a set of PPG signals, the set of PPG signals acquired using the one or more light emitters and the one or more light detectors disposed in or on a portion of a wrist-worn wearable device,
separate the set of PPG signals into at least a physiological portion of the set of PPG signals and a motion artifact portion of the set of PPG signals, and
identify at least one hand or finger gesture of the wearer of the wrist-worn wearable device based at least in part on the motion artifact portion of the set of PPG signals.

12. The wrist-worn wearable device of claim 11, further comprising at least one of: an inertial measurement unit; at least one sensor configured to obtain impedance plethysmography signals; or one or more electromyography (EMG) electrodes, wherein the at least one hand or finger gesture is determined based on the inertial measurement unit, data obtained using the at least one sensor configured to obtain impedance plethysmography signals, or EMG signals obtained using the one or more EMG electrodes.

13. The wrist-worn wearable device of claim 11 or claim 12, wherein the wrist-worn wearable device is communicatively coupled to an augmented reality (AR) or virtual reality (VR) headset, and wherein the at least one hand or finger gesture is used to control a functionality of the AR or VR headset.

14. The wrist-worn wearable device of any one of claims 11 to 13, wherein at least one light emitter and light detector pair is spatially separated from another light emitter and light detector pair.

15. The wrist-worn wearable device of any one of claims 11 to 14, and one or more of the following:
(i) wherein the one or more light emitters and the one or more light detectors are spatially separated and disposed along a back portion of a capsule of the wrist-worn wearable device;
(ii) wherein light emitters of the one or more light emitters are configured to emit light toward skin of a wearer of the wrist-worn wearable device using at least two different wavelengths of light.
